Europäisches Patentamt

European Patent Office

Office européen des brevets

(11) Veröffentlichungsnummer: **0 126 256**
A1

(12) **EUROPÄISCHE PATENTANMELDUNG**

(21) Anmeldenummer: 84103833.4

(22) Anmeldetag: 06.04.84

(51) Int. Cl.³: **A 61 F 13/10**

(30) Priorität: 27.04.83 DE 8312360 U

(43) Veröffentlichungstag der Anmeldung: 28.11.84
Patentblatt 84/48

(84) Benannte Vertragsstaaten: AT BE CH DE FR LI NL SE

(71) Anmelder: Hildebrandt, Hans-Dietrich, Dr. med.,
Hohlesteinweg 16, D-3501 Ahnatal (DE)

(72) Erfinder: Hildebrandt, Hans-Dietrich, Dr. med.,
Hohlesteinweg 16, D-3501 Ahnatal (DE)

(74) Vertreter: Freiherr von Schorlemer, Reinfried,
Brüder-Grimm-Platz 4, D-3500 Kassel (DE)

(54) Epicondylitis-Bandage.

(57) Die Bandage (1) besteht aus einem um den Unterarm zu legenden Band mit einem Funktionsabschnitt (2) und an diesem befestigten Verschlußelementen (5, 6, 7, 8). Der Funktionsabschnitt weist auf seiner Innenseite einen zur Beeinflußung einer zu behandelnden Muskelgruppe bestimmten Wulst (12) mit planer Oberfläche (13) auf.

EP 0 126 256 A1

Epicondylitis-Bandage

Die Erfindung betrifft eine Epicondylitis-Bandage der im
Oberbegriff des Anspruchs 1 definierten Gattung.

Epicondylitis, häufig auch als "Tennisarm" bezeichnet, entsteht durch Überbeanspruchung einer Muskelgruppe im Ellenbogenbereich und hat heftige Schmerzen bei Druck und Belastung zur Folge. Außer bei Sportlern wie Tennis-, Tischtennis-, Badminton- oder Golfspielern tritt dieses Leiden
auch bei Personen auf, die infolge beruflicher oder privater Tätigkeiten die genannte Muskelgruppe häufig überlasten.

Zur Linderung der Schmerzen sind Epicondylitis-Bandagen der
eingangs bezeichneten Gattung bekannt (US-PS 3 877, 426,
DE-Gm 81 09 073), mittels derer der Muskelansatz der betreffenden Muskelgruppe künstlich vorverlegt wird, um
bei Benutzung der Hand bzw. des Arms entlastet zu werden.
Als störend wird dabei empfunden, daß der durch das Festziehen der Bandage ausgeübte Druck gleichmäßig auf diejenigen Teile der Unterarms verteilt wird, die vom Funktionsabschnitt und den Verschlußelementen, z.B. bendförmigen,
zur Bildung eines Klettenverschlusses bestimmten Streifen,
bedeckt werden. Es besteht dabei nämlich die Gefahr, daß
die Bandage vom Benutzer entweder zu lose angelegt wird
und dann keinen wesentlichen Einfluß auf die erkrankte
Muskelgruppe hat oder aber zu fest angezogen wird und
dann nicht nur auf die zu beeinflussende Muskelgruppe einwirkt, d.h. unnötig auch an sich leistungsfähige Muskelgruppen entlastet, was zu deren Minderbelastbarkeit bis zur
Inaktivitätsatrophie führen kann und aus medizinischer Sicht
nicht vertretbar ist. Eine dosierte Behandlung der jeweils
betroffenen Muskelgruppe ist mit derartigen Bandagen nicht
möglich. Außerdem kann bei zu festem Anlegen der Bandage
der Rückfluß des venösen Blutes gestört werden, d.h. ein
Blutstau entstehen, was ein längeres Tragen der Bandage
unmöglich macht.

Neben derartigen Epicondylitis-Bandagen sind Epicondylitis-
Klammern bekannt (DE-PS 26 35 426). Diese enthalten anstelle
des bandförmigen Funktionsabschnitts eine halbringförmige,
federnde Klammer, deren freie Enden als Druckplatten mit
unterschiedlich großen Druckflächen ausgebildet sind,die
sich beim Tragen an jeweils zwei diametral gegenüberliegende
Muskelgruppen des Unterarms anlegen. Dadurch werden mit derartigen Klammern wie bei Anwendung bekannter Bandagen stets
auch an sich leistungsfähige Muskelgruppen unnötig entlastet.
Außerdem ist der individuell ausgeübte Druck hauptsächlich
durch die der Klammer innewohnende Federkraft, die Größe der
Druckplatten und die individuelle Unterarmgröße bzw. die
dadurch beim Tragen bedingte Spreizung der Klammer vorgegeben, was eine individuelle Druckdosierung unmöglich macht.
Störende Einflüsse auf den Rückfluß des venösen Blutes sollen bei Anwendung der Klammer zwar dadurch vermieden werden,
daß der die Druckplatten verbindende Bogen den Unterarm beim
Tragen der Klammer nicht berührt. Diese Forderung ist jedoch
kaum realisierbar, weil ein sinnvolles Tragen der Klammer
nur bei festgezogenen Verschlußelementen möglich ist und
ein Festziehen derselben automatisch ein Heranziehen des
Klammerbügels an den Unterarm zur Folge hat. Da schließlich
die Druckplatten unterschiedliche Größen aufweisen, wobei
die größere Druckplatte der erkrankten Muskelgruppe zugeordnet ist, wird in der Regel die gesunde Muskelgruppe
stärker als die erkrankte Muskelgruppe beeinflußt, was beim
Tragen der Klammer als unbequem und störend empfunden wird und sogar Schmerzen verursachen kann, die ein längers Tragen der Klammer nicht zulassen.

Der Erfindung liegt daher die Aufgabe zugrunde, die Epicondylitis-Bandage der eingangs bezeichneten Gattung so auszubilden, daß nur die individuell erkrankte Muskelgruppe
gezielt und dosierbar beeinflußt wird, während die jeweils
anderen Muskelgruppen weitgehend unbeeinflußt bleiben. Außerdem soll vermieden werden, daß die Bandage zu fest angezogen
werden muß und dadurch der Rückfluß des venösen Blutes gestört wird.

Zur Lösung dieser Aufgabe sind die kennzeichnenden Merkmale
des Anspruchs 1 vorgesehen.

Weitere vorteilhafte Merkmale ergeben sich aus den Unteransprüchen.

Die Erfindung bringt den Vorteil mit sich, daß mittels des
Wulstes eine vorgewählte Muskelgruppe gezielt beeinflußt
werden kann. Dadurch, daß der Wulst eine planare Oberfläche
aufweist, wird gleichzeitig eine im wesentlichen gleichförmige Beeinflussung der zu behandelnden Muskelgruppe über
deren ganze Breite erzielt. Die Größe des im Einzelfall
auszuübenden Drucks ist individuell einstellbar bzw. dosierbar, weil beim Festziehen der Verschlußelemente eine mehr
oder weniger starke Druckerhöhung vor allem im Wulstbereich
stattfindet, während in den übrigen Bereichen des Funktionsabschnitts und der bandförmigen Verschlußelemente nur eine
geringfügige Druckerhöhung eintritt, was gleichzeitig die
Gefahr von Durchblutungsstörungen erheblich verringert.

Die Erfindung wird nachfolgend in Verbindung mit der beiliegenden Zeichnung an einem Ausführungsbeispiel näher erläutert. Es zeigen:

Fig. 1 eine Draufsicht auf eine erfindungsgemäße Epicondylitis-Bandage im abgerollten Zustand; und

Fig. 2 eine Draufsicht auf die Bandage nach Fig. 1.

Die erfindungsgemäße Epicondylitis-Bandage 1 weist einen Funktionsabschnitt 2 auf, der in bekannter Weise (DE-Gm 81 09 073) von zwei bogenförmigen, an die Anatomie des Unterarms angepaßten Längsrändern 3 und 4 begrenzt ist. Unter einer an die Anatomie des Unterarms angepaßten Form wird hierbei verstanden, daß der Funktionsabschnitt 2 wie der dicht unterhalb des Ellenbogens befindliche Teil des Unterarms im Gebrauchszustand etwa längs einer Kegelstumpffläche verläuft und sich daher fest und gleichförmig dem Unterarm anschmiegt. An den Enden des Funktionsabschnitts 2 sind Verschlußelemente vorgesehen, die beispielsweise aus bandförmigen Streifen 5 und 6 bestehen, von denen der eine Schichten 7 zur Bildung eines Klettenverschlusses aufweist, während der andere an seinem freien Ende mit einem Ring bzw. einer Öse 8 od. dgl. versehen ist, durch die der Streifen 6 durchgezogen und dann auf sich selbst zurückgefaltet wird.

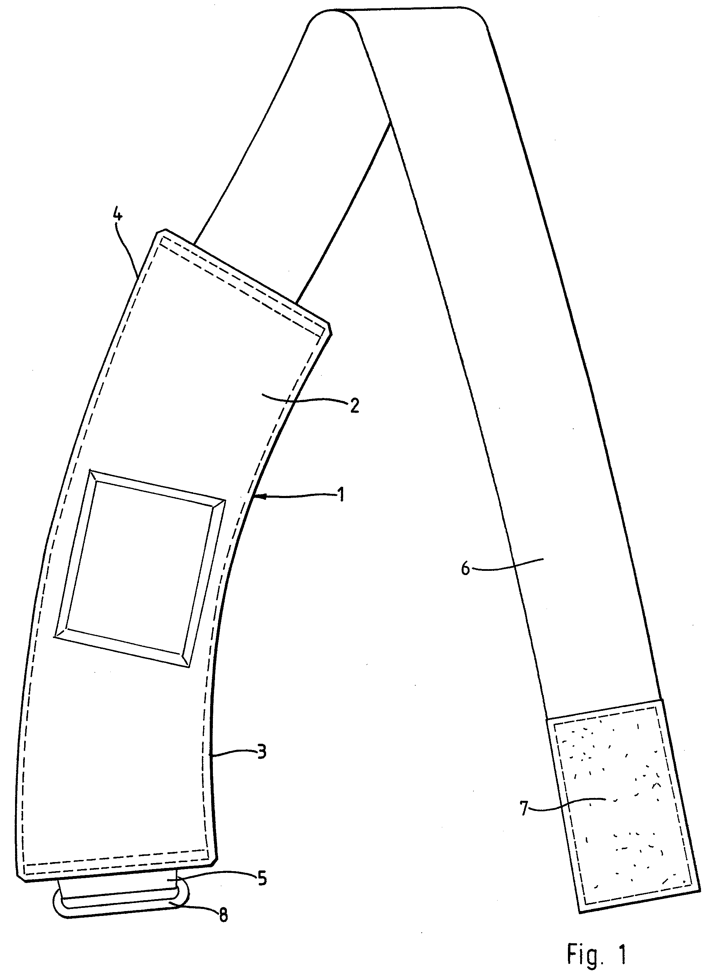

Der Funktionsabschnitt 2 braucht nicht exakt wie ein Kegelstumpfmantel geformt sein, weil bereits die durch den bogenförmigen Verlauf der Längsränder 3 und 4 erzielte Form ein Rutschen der Bandage am Unterarm nahezu ausschließt, wenn der Krümmungsradius der Längsränder 3 und 4 unter Berücksichtigung der Anatomie des Unterarms ausreichend groß gewählt ist. Bei einer praktischen Ausführungsform beträgt die Länge des oberen Längsrandes z.B. etwa 17 cm, die Länge des unteren Längsrandes 3 etwa 14,5 cm, die Höhe $\underline{h}$ des Funktionsabschnitts 2 etwa 4,5 cm und der Radius $\underline{R}$ der bei-den Längsränder 3 und 4 etwa 30 bis 40 cm. Die Streifen 5 und 6 schließen sich an den beiden Enden des Funktionsabschnitts 2 etwa tangential an und könnten entgegen Fig. 1 ebenfalls anatomiegerecht geformt sein.

Obwohl eine Bandage 1 mit den angegebenen Dimensionen bei geeigneter Länge der Streifen 5 und 6 praktisch für Unterarme jeder Größe brauchbar ist und dabei zur Behandlung sowohl der Epicondylitis humeri radialis als auch der Epicondylitis humeri ulnaris benutzt werden kann, wird die erfindungsgemäße Bandage 1 zweckmäßig in zwei oder mehr Größen hergestellt und geliefert, damit auch bei besonders stark oder schwach entwickelten Unterarmen der erwünschte feste Sitz gewährleistet ist. Außerdem kann entsprechend Fig. 1 vorgesehen sein, den beiden Längsrändern 3 und 4 unterschiedliche Krümmungsradien zu geben, so daß beispielsweise die Höhe $\underline{h}$ des Funktionsabschnitts 2 im mittleren Bereich etwas größer als an den beiden Enden ist.

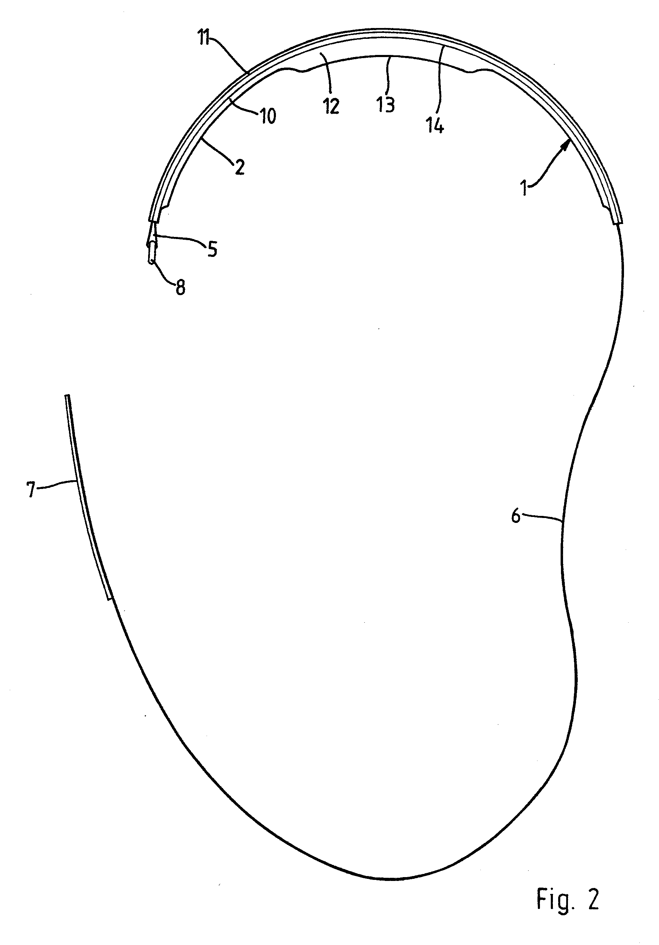

Als Materialien für den Funktionsabschnitt eignen sich beispielsweise Leder, Kunstleder und ein von der Fa. Heinz Ziegenbein KG, 7000 Stuttgart unter der Bezeichnung "Molliskin" vertriebenes Material, das aufgrund seiner veloursartigen, rauhen Oberfläche die Haftung der Bandage 1 am Arm zusätzlich fördert. Vorzugsweise besteht der Funktionsabschnitt 2 aus zwei taschen- bzw. beutelförmig längs einer Naht 9 zusammengenähten Schichten 10 und 11, wobei die innere, am Arm anliegende Schicht 10 z.B. aus Kunstleder und die Schicht 11 aus Velours hergestellt ist. Die beiden Streifen 5 und 6 können dabei entweder am Funktionsabschnitt 2 beispielsweise durch Nähen befestigt sein oder die Endabschnitte eines durchgehenden Bandes bilden, dessen Mittelteil mit einem Materialabschnitt derart umwikkelt und beispielsweise vernäht ist, daß sich die Form nach Fig. 1 und 2 ergibt. Auch andere Ausführungsformen sind denkbar. Die Streifen 5 und 6 bestehen zweckmäßig aus einem normalen Gurtband, das bei Anwendung eines durchgehenden Bandes in seinem den Funktionsabschnitt 2 tragenden Bereich zweckmäßig entsprechend bogenförmig gestaltet ist. Alternativ könnten der Funktionsabschnitt 2 und die Streifen 5,6 aus einem Stück bestehen und beispielsweise aus einem entsprechend zugeschnittenen Materialstreifen hergestellt sein.

Als Verschlußelemente können andere als die dargestellten, einen Klettenverschluß bildenden Verschlußelemente vorgesehen werden, um die Bandage am Unterarm zu fixieren. Der freie Bügel des Rings 8 kann beispielsweise eine Rolle tragen, die das Festziehen des in den Ring 8 eingelegten Streifens 6 beim Anlagen der Bandage 1 erleichtert.

Der Funktionsabschnitt 2 ist auf seiner Innenseite, die beim Tragen der Bandage am Arm anliegt, mit einem zur gezielten Beeinflussung einer erkrankten Muskelgruppe bestimmten Wulst 12 versehen, der eine plane Oberfläche 13 aufweist. Die Länge des Wulstes 12 sollte so gewählt sein, daß sich die planare Oberfläche über die gesamte Breite der jeweils zu behandelnden Muskelgruppe, z.B. der Muskelgruppe der Handgelenk- und Fingerstrecker oder Handgelenk- und Fingerbeuger, erstrecken kann. Bei einer beispielsweisen Ausführungsform ist ein Wulst mit einer Länge von etwa 4,5 cm, einer Breite von etwa 3,5 cm und einer Höhe von einigen Millimetern vorgesehen. Der Wulst 12 ist außerdem zweckmäßig in einem mittleren Bereich des Funktionsabschnitts 2 angeordnet und aus einem Füllstück hergestellt, das in den kissen- oder beutelförmigen Funktionsabschnitt 2 eingelegt ist. Als Material wird für den Wulst 12 vorzugsweise ein halbelastisches Material gewählt, z.B. ein formbeständiger Schaumstoff, das einerseits nicht zu hart ist, um beim Tragen der Bandage als störend empfunden zu werden, andererseits jedoch nicht zu nachgiebig ist und dadurch seine Funktion zur Konzentration des Drucks auf die betroffene Muskelgruppe verliert.

Bei einer bevorzugten Ausführungsform ist der Funktionsabschnitt 2 außerdem mit einer nicht näher dargestellten, in dessen Längsrichtung gekrümmten, scheiben- bzw. plattenförmigen Einlage aus einem elastischen Material, z.B. Kunststoff, versehen, die etwa in der durch eine Linie 14 angedeuteten Mittelebene des Funktionsabschnitts 2 verläuft und etwa dieselbe Länge und Breite wie dieser, jedoch eine

geringeeDicke besitzt. Diese Einlage dient einerseits dem
Zweck, den Funktionsabschnitt 2 zu versteifen und dem den
Wulst 12 bildenden Füllstück als Auflage zu dienen, das
zwischen der Einlage und der inneren Schicht 10 des Funktionsabschnitts angeordnet ist. Andererseits soll die Einlage dem Funktionsabschnitt in dessen Nichtgebrauchslage
etwa die aus Fig. 2 ersichtliche bogenförmige Gestalt geben,
die das Anlegen der Bandage und das exakte Plazieren des
Wulstes 12 erleichtert, ohne dadurch eine Klammerwirkung
(DE-PS 26 35 426) zu erzielen, die unerwünscht ist. Die
Einlage besteht außerdem aus einem so elastischen Material,
daß sie beim Anlegen der Bandage ein anatomiegerechtes Anschmiegen der Bandage an den Unterarm gestattet.

Die erfindungsgemäße Epicondylitis-Bandage kann wahlweise
zur Beeinflussung der Muskelgruppe der Handgelenk- und
Fingerstrecker oder der Handgelenk- und Fingerbeuger verwendet werden. Dabei ergibt sich der Vorteil, daß die jeweils andere Muskelgruppe nicht wesentlich beeinflußt wird.
Eine Dosierung des vom Wulst 12 ausgeübten Drucks wird durch
mehr oder weniger starkes Festziehen der Bandage mittels der
Verschlußelemente bzw. der Streifen 5 und 6 vorgenommen.
Weil dadurch der Wulst 12 mehr oder weniger stark gegen
die zu behandelnde Muskelgruppe gedrückt wird, findet
außerdem eine Konzentration des Drucks in diesem Bereich
statt, während der Druck in den übrigen Bereichen gleichförmig verteilt wird und vergleichsweise gering ist, so
daß Blutstauungen nicht auftreten können.

Patentanwalt
Diplom-Physiker
**Reinfried Frhr. v. Schorlem**

D-3500 Kassel
Brüder-Grimm-Platz 4
Telefon (0561) 15335

0126256

D 5327

Dr. Hans-Dietrich Hildebrandt, 3501 Ahnatal

Schutzansprüche

1) Epicondylitis-Bandage, bestehend aus einem um den Unterarm zu legenden Band mit einem Funktionsabschnitt und an diesem befestigten Verschlußelementen, dadurch gekennzeichnet, daß der Funktionsabschnitt (2) auf seiner Innenseite einen zur Beeinflussung einer zu behandelnden Muskelgruppe bestimmten Wulst (12) mit planer Oberfläche aufweist.

2) Bandage nach Anspruch 1, dadurch gekennzeichnet, daß der Wulst (12) aus einem in den Funktionsabschnitt (2) eingelegten Füllstück besteht.

3) Bandage nach Anspruch 1 oder 2, dadurch gekennzeichnet, daß der Funktionsabschnitt (2) eine diesen versteifende und in dessen Längsrichtung gekrümmte Einlage aus einem elastischen Material aufweist.

4) Bandage nach wenigstens einem der Ansprüche 1 bis 3, dadurch gekennzeichnet, daß wenigstens der Funktionsabschnitt (2) eine an die Anatomie des Unterarms angepaßte Form besitzt.

0126256

5) Bandage nach Anspruch 4, dadurch gekennzeichnet, daß
der Funktionsabschnitt (2) längs der Abwicklung eines Kegelstumpfmantels verläuft.

6) Bandage nach wenigstens einem der Ansprüche 1 bis 5,
dadurch gekennzeichnet, daß der Funktionsabschnitt (2) aus
einem rutschfesten Material besteht.

7) Bandage nach wenigstens einem der Ansprüche 1 bis 6,
dadurch gekennzeichnet, daß die Länge des Funktionsabschnitts (2) etwa einem halben Armumfang entspricht.

Fig. 1

Fig. 2

# EUROPÄISCHER RECHERCHENBERICHT

**Europäisches Patentamt**

0126256

Nummer der Anmeldung

EP 84 10 3833

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | US-A-4 243 028 (PUYANA)<br>* Ansprüche 1, 5; Spalte 1, Zeile 61 - Spalte 2, Zeile 6; Figuren 1-4 * | 1,2 | A 61 F 13/10 |
| A | | 3 | |
| Y | US-A-3 970 081 (APPLEGATE)<br>* Anspruch 1; Spalte 4, Zeilen 12-23; Figuren 1, 5 * | 1,2 | |
| A | | 4 | |
| Y | DE-U-8 127 009 (BORT)<br>* Ganzes Dokument * | 1,2 | |
| A | | 4,5 | RECHERCHIERTE SACHGEBIETE (Int. Cl. 3)<br><br>A 61 F 13/00 |
| A | US-A-4 027 666 (MARX)<br>* Anspruch 1; Figuren 1, 2 * | 3,7 | |
| A | US-A-3 318 305 (SCHULTZ) | | |
| A | US-A-3 942 525 (DRAGAN) | | |
| D,A | DE-C-2 635 426 (BEIGE et al.) | | |
| | --- -/- | | |

Der vorliegende Recherchenbericht wurde für alle Patentansprüche erstellt.

| Recherchenort<br>BERLIN | Abschlußdatum der Recherche<br>20-07-1984 | Prüfer<br>KANAL P K |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| D,A | DE-U-8 109 073 (HILDEBRANDT) | | |
| | --- | | |
| D,A | US-A-3 877 426 (NIRSCHL) | | |
| | ----- | | |

RECHERCHIERTE
SACHGEBIETE (Int. Cl. ³)

Der vorliegende Recherchenbericht wurde für alle Patentansprüche erstellt.

| Recherchenort BERLIN | Abschlußdatum der Recherche 20-07-1984 | Prüfer KANAL P K |
|---|---|---|